# EUROPEAN PATENT APPLICATION

(11) **EP 4 442 285 A1**
(43) Date of publication of application: **09.10.2024**
(21) Application number: 22901284.4
(22) Date of filing: 29.11.2022
(51) Int. Cl.: A61K 51/08, A61K 47/10, A61K 47/20, A61K 47/26, A61K 47/36, A61P 43/00

(54) **STABILIZED RADIOPHARMACEUTICAL COMPOSITION**

(30) Priority: 30.11.2021 JP 2021194992
(71) Applicant: Nihon Medi-Physics Co., Ltd., Tokyo 136-0075 (JP)
(72) Inventor: ICHIKAWA, Hiroaki, Tokyo 136-0075 (JP); NAKAMURA, Shunsuke, Tokyo 136-0075 (JP); TSUSHIMA, Michihiko, Tokyo 136-0075 (JP); SATO, Takumi, Tokyo 136-0075 (JP); KISHIMOTO, Satoshi, Tokyo 136-0075 (JP); OTSUKA, Yuta, Tokyo 136-0075 (JP)
(74) Representative: TBK
(86) International application number: PCT/JP2022/043913
(87) International publication number: WO 2023/100852

(57) **Abstract**

An object of the present invention is to provide a stable radiopharmaceutical composition in which the decrease in the radiochemical purity over time is suppressed even in the case where the radioactivity concentration is high immediately after production.

The present invention related to a stabilized radiopharmaceutical composition comprising a polypeptide labeled with a radionuclide and having a group selected from the group consisting of a thioether group and a thiol group, as an active ingredient,
a solubilizing agent having an ethylene glycol group,
a solvent comprising water, and
a stabilizer selected from the group consisting of a sulfur-containing amino acid or a salt thereof, a sulfur-containing amino acid derivative or a salt thereof, and a polysaccharide.

## Description

### [Technical Field]

The present invention relates to a stabilized radiopharmaceutical composition.

### [Background Art]

The stability of active ingredients is important in the development of pharmaceuticals.

One of the causes of decreased stability of radiopharmaceuticals is that the radiation emitted by radionuclides causes radiolysis, which destroys the molecular structure of the active ingredient and the chemical bonds of other ingredients.

Therefore, stabilizers are often used in radiopharmaceutical compositions.

Patent Literature 1 describes that for an antibody labeled with ¹³¹I (L19-SIP), at the ¹³¹I radioactivity concentration around 84 to 104 MBq/mL, maltose, methionine, gentisic acid, ascorbic acid, trehalose, benzyl alcohol and thiosulphate have a stabilizing effect when added (Tables 5 and 6), and at the ¹³¹I radioactivity concentration of 370 mBq/mL, ascorbic acid and thiosulphate have a stabilizing effect when added (Table 10) .

Patent Literature 2 describes that for a complex of a methionine-containing peptide containing DOTA (1,4,7,10-tetraazacyclododecane-1,4,7,10-tetraacetic acid) and ¹⁷⁷Lu, at the ¹⁷⁷Lu radioactivity concentration less than 740 MBq/mL, cysteine and methionine have a stabilizing effect (Table 5), but at the higher ¹⁷⁷Lu radioactivity concentrations of 925 MBq/mL, cysteine and methionine have no stabilizing effect when added alone (Table 6).

Non Patent Literature 1, although not related to radiopharmaceuticals, describes that peroxides derived from polysorbate 80, a non-surfactant widely used as a solubilizing agent for pharmaceuticals, cause oxidation (oxidation of a methionine residue) of an antibody (IL-2 muteins) (Fig. 5) and that the addition of glutathione and storage under vacuum conditions show a stabilizing effect (Fig. 7).

### [Citation List]

### [Patent Literature]

[Patent Literature 1]
   WO 2011/147762 pamphlet
[Patent Literature 2]
   JP 2006-528644

### [Non Patent Literature]

[Non Patent Literature 1]
Journal of Pharmaceutical Sciences, Vol.91, No.10, pages 2252-2264, 2002

### [Summary of Invention]

An object of the present invention is to suppress a decrease in radiochemical purity over time in a radiopharmaceutical composition comprising a radioactive polypeptide having a specific group as an active ingredient.

In developing a radiopharmaceutical composition comprising a radioactive polypeptide having a thioether group and/or a thiol group as an active ingredient, the present inventors have found that the addition of a solubilizing agent is necessary to prevent the radioactive polypeptide from adsorbing to a container or administration equipment. They have also found that the addition of a solubilizing agent causes a new problem in that the radiochemical purity of the radioactive polypeptide is reduced.

The present inventors have conducted intensive studies in an attempt to solve such problem inherent in the addition of a solubilizing agent and found that the problem can be solved by adding a specific stabilizer, which resulted in the completion of the present invention.

Accordingly, one embodiment of the present invention provides a stabilized radiopharmaceutical composition comprising
a polypeptide labeled with a radionuclide and having a group selected from the group consisting of a thioether group and a thiol group, as an active ingredient,
a solubilizing agent having an ethylene glycol group,
a solvent comprising water, and
a stabilizer selected from the group consisting of a sulfur-containing amino acid or a salt thereof, a sulfur-containing amino acid derivative or a salt thereof, and a polysaccharide.

According to the present invention, even in a formulation where a solubilizing agent having an ethylene glycol group is present, certain stabilizers can suppress the decrease in the radiochemical purity of a radioactive polypeptide having a thioether group and/or a thiol group over time, thus a stabilized radiopharmaceutical composition can be provided.

### [Brief Description of Drawings]

[Fig. 1]

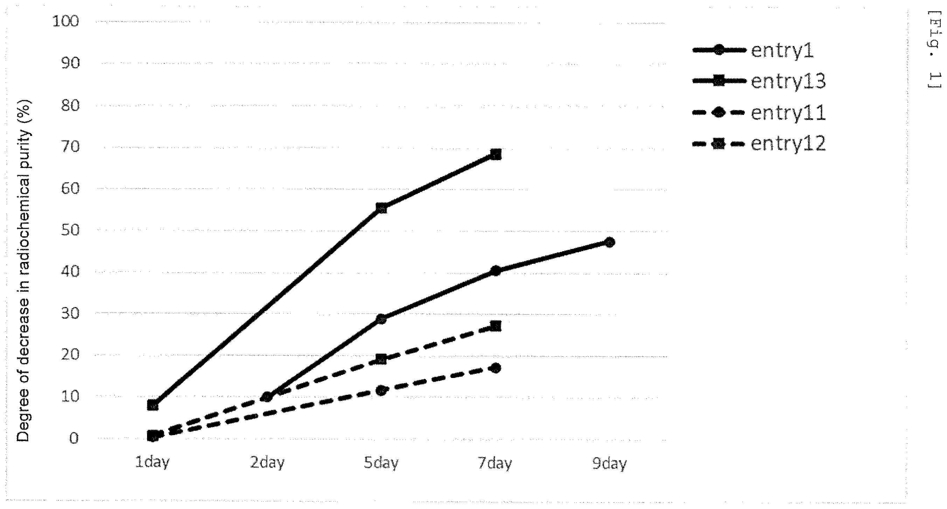

   Fig. 1 shows the decrease in the radiochemical purity of the ⁸⁹Zr-labeled product over time when polysorbate 80 and/or ethanol are added.
[Fig. 2]

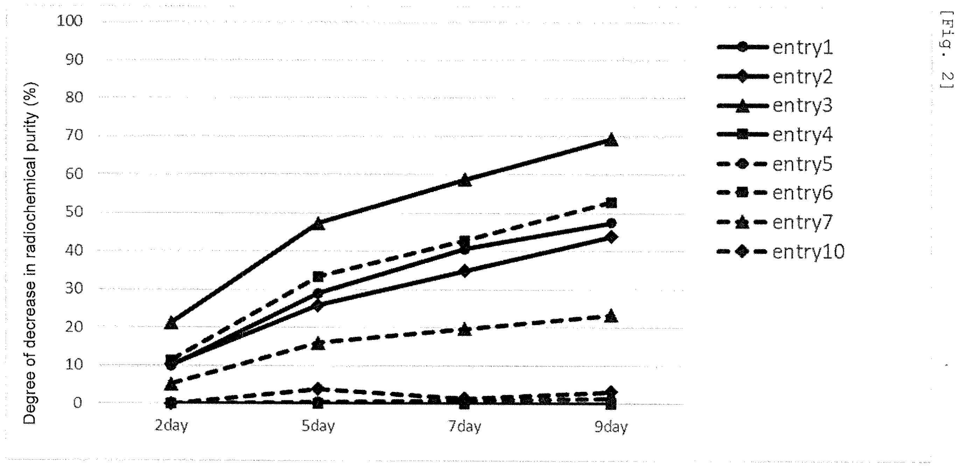

   Fig.2 shows the decrease in the radiochemical purity of the ⁸⁹Zr-labeled product over time when various reagents are added.
[Fig. 3]

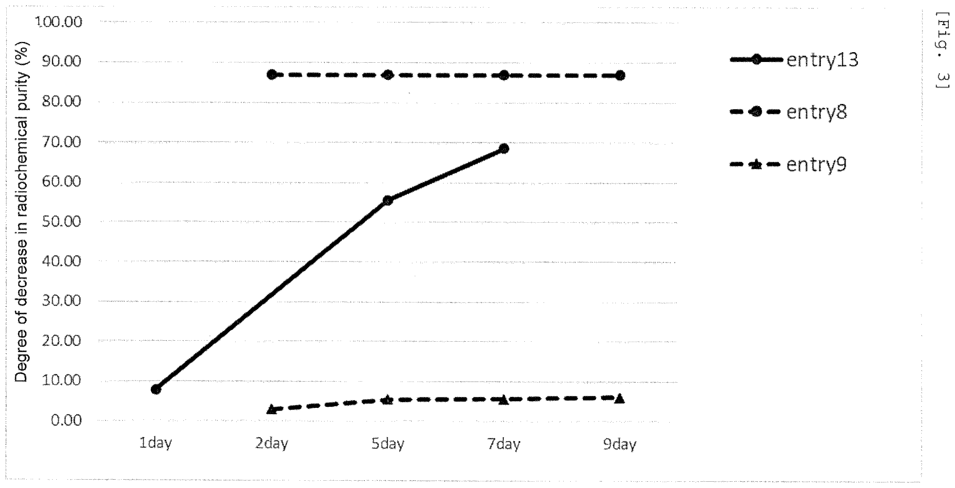

   Fig.3 shows the decrease in the radiochemical purity of the ⁸⁹Zr-labeled product over time when polysorbate 80 or macrogol4000 and/or methionine are added.

### [Description of Embodiments]

The present invention is described in detail below.

The stabilized radiopharmaceutical composition of the present invention comprising a polypeptide labeled with a radionuclide and having a group selected from the group consisting of a thioether group and a thiol group (hereinafter also to be referred to as "radioactive polypeptide") as an active ingredient, a solubilizing agent having an ethylene glycol group,
a solvent comprising water, and
a stabilizer selected from the group consisting of a sulfur-containing amino acid or a salt thereof, a sulfur-containing amino acid derivative or a salt thereof, and a polysaccharide.

### (1) Radioactive polypeptide

The radioactive polypeptide in the present invention is a polypeptide labeled with a radionuclide and having a group selected from the group consisting of a thioether group and a thiol group.

### (1-1) Radionuclide

The radionuclide contained in the radioactive polypeptide in the present invention is a radionuclide that emits α-ray, a radionuclide that emits β-ray, a radionuclide that emits positron, or a radionuclide that emits γ-ray. When the radioactive polypeptide in the present invention is used for cancer therapy, it is preferable to use a radionuclide that emits α-ray or a radionuclide that emits β-ray. When the radioactive polypeptide in the present invention is used for cancer diagnosis or detection, it is preferable to use a radionuclide that emits positron, or a radionuclide that emits γ-ray. In the present invention, it is preferable to use a metal radionuclide as the radionuclide. Examples of the radionuclide that emits α-ray include ²¹²Bi, ²¹³Bi, ²²⁵Ac, ²²⁷Th and the like. Examples of the radionuclide that emits β-ray include ⁶⁴Cu, ⁹⁰Y, ¹⁷⁷Lu and the like. Examples of the radionuclide that emits positron include ¹⁸F, ⁶⁴Cu, ⁶⁸Ga, ^{8S}Y, ⁸⁹Zr and the like. Examples of the radionuclide that emits γ-ray include ^{99m}Tc, ⁶⁷Ga, ¹¹¹In and the like. The radionuclide contained in the radioactive polypeptide in the present invention is preferably selected from the group consisting of ⁸⁹Zr, ²²⁵Ac, ¹⁷⁷Lu, ¹¹¹In, ⁹⁰Y, ⁶⁷Ga and ⁶⁸Ga.

The radionuclide may be introduced into a polypeptide having a group selected from the group consisting of a thioether group and a thiol group, either by a covalent bond or by chelation. In the case of the embodiment introduced by chelation, a metal radionuclide or a radiohalogenated metal (for example, Al¹⁸F) is used as the radionuclide. For this embodiment, the description below regarding a radioconjugate containing a polypeptide and a chelate complex can be referred to.

### (1-2) Polypeptide

The polypeptide in the present invention is a polypeptide having a group selected from the group consisting of a thioether group and a thiol group, and is a polypeptide having a chemical structure for expressing tropism toward a target organ or tissue in a living body or specificity for a target molecule, as a targeting agent for radiotherapy or radiodiagnosis. The polypeptide in the present invention may be cyclic or chain. The polypeptide in the present invention has a molecular weight of not less than 500, preferably 500 to 10000, and does not include antibodies.

As used herein, the "thioether group" is represented by formula -S-R wherein R is an alkyl group (preferably having 1 to 10 carbon atoms), an aryl group (preferably having 6 to 10 carbon atoms), a cycloalkyl group (preferably having 3 to 10 carbon atoms) or an aralkyl group (preferably having 7 to 10 carbon atoms), and specific examples thereof include methylthio, phenylthio, benzylthio, cyclohexylthio and the like.

In the polypeptide in the present invention, the positions of the thioether group and thiol group are not particularly limited as long as the polypeptide has the above chemical structure. For example, they may be a target molecule recognition site composed of a specific amino acid sequence, or may be contained in a linker site other than the target molecule recognition site (for example, linker (L) in a conjugate containing a polypeptide and a chelate complex, described later).

Examples of the group consisting of a thioether group and a thiol group possessed by the polypeptide in the present invention include thioether groups and thiol groups possessed by natural sulfur-containing amino acids such as cysteine, methionine and homocysteine; and thioether groups and thiol groups possessed by non-natural sulfur-containing amino acids. These sulfur-containing amino acid may be a D-form or a L-form. Furthermore, the thiol group possessed by the polypeptide may be derivatized. For example, the two thiol groups possessed by the polypeptide may be linked to each other to form a disulfide bond; the thiol group possessed by the polypeptide may be taken together with another functional group to form a bond (for example, the thiol group possessed by the polypeptide is linked to a chloroacetyl group to form a thioether bond (-CO-CH₂-S-)). Such derivatized thiol groups are also included in the group consisting of a thioether group and a thiol group possessed by the polypeptide in the present invention.

The radioactive polypeptide in the present invention includes a polypeptide having a group selected from the group consisting of a thioether group and a thiol group into which a radionuclide is covalently introduced; and a conjugate containing a polypeptide having a group selected from the group consisting of a thioether group and a thiol group and a complex formed from a chelating agent and a radionuclide (chelate complex). In the present invention, the radioactive polypeptide is preferably a radioconjugate containing a polypeptide having a group selected from the group consisting of a thioether group and a thiol group and a chelate complex (hereinafter to be also referred to as "radioconjugate"), which is described in detail below.

### (1-3) Radioconjugate

In the present invention, the chelating agent is not particularly limited as long as it has a site capable of chelating a radionuclide in its structure. Examples of the chelating agent include
CB-TE2A (1,4,8,11-Tetraazabicyclo[6.6.2]hexadecane-4,11-diacetic acid),
CDTA (Cyclohexane-trans-1,2-diamine tetraacetic acid), CDTPA (4-cyano-4-[[(dodecylthio)thioxomethyl]thio]-pentanoic acid),
DOTA (1,4,7,10-Tetraazacyclododecane-1,4,7,10-tetraacetic acid),
DOTMA ((1R,4R,7R,10R)-α,α',α",α‴-tetramethyl-1,4,7,10-tetraazacyclododecane-1,4,7,10-tetraacetic acid),
DOTAM (1,4,7,10-tetrakis(carbamoylmethyl)-1,4,7,10-tetraazacyclododecane),
DOTPA (1,4,7,10-tetraazacyclododecane-1,4,7,10-tetrapropionic acid),
1,4,7,10-tetrakis(pyridin-2-ylmethyl)-1,4,7,10-tetraazacyclododecane (Lpy),
DOTA-GA (α-(2-Carboxyethyl)-1,4,7,10-tetraazacyclododecane-1,4,7,10-tetraacetic acid),
DOTP (((1,4,7,10-Tetraazacyclododecane-1,4,7,10-tetrayl)tetrakis(methylene))tetraphosphonic acid),
DOTMP (1,4,7,10-Tetraazacyclododecane-1,4,7,10-tetrakis(methylenephosphonic acid)),
DOTA-4AMP (1,4,7,10-tetraazacyclododecane-1,4,7,10-tetrakis(acetamidomethylenephosphonic acid)),
D02P (Tetraazacyclododecane dimethanephosphonic acid), Deferoxamine (DFO),
DTPA (Glycine, N,N-bis[2-[bis(carboxymethyl)amino]ethyl]-),
CHX-A"-DTPA (2,2'-((2-(((1S,2R)-2-(bis(carboxymethyl)amino)cyclohexyl)(carboxymethyl)amino) ethyl)azanediyl)diacetic acid),
DTPA-BMA (5,8-Bis(carboxymethyl)-11-[2-(methylamino)-2-oxoethyl]-3-oxo-2,5,8,11-tetraazatridecan-13-oic acid), EDTA (2,2',2",2‴-(ethane-1,2-diylbis(azanetriyl))tetraacetic acid),
NOTA (1,4,7-Triazacyclononane-1,4,7-triacetic acid), NOTP (1,4,7-Triazacyclononane-1,4,7-triyltris(methylenephosphonic acid)),
TETPA (1,4,8,11-tetraazacyclotetradecane-1,4,8,11-tetrapropionic acid),
TETA (1,4,8,11-Tetraazacyclotetradecane-N,N',N",N‴-tetraacetic acid),
TTHA (3,6,9,12-Tetrakis(carboxymethyl)-3,6,9,12-tetraazatetradecanedioic acid),
HEHA (1,2,7,10,13-hexaazacyclooctadecane-1,4,7,10,13,16-hexaacetic acid),
1,2-HOPO (N,N',N",N‴-tetra(1,2-dihydro-1-hydroxy-2-oxopyridine-6-carbonyl)-1,5,10,14-tetraazatetradecane), PEPA (1,4,7,10,13-pentaazacyclopentadecane-N,N',N",N‴,Nʺʺ-pentaacetic acid),
H4octapa (N,N'-bis(6-carboxy-2-pyridylmethyl)-ethylenediamine-N,N'-diacetic acid),
H2bispa2 (6,6'-({9-hydroxy-1,5-bis(methoxycarbonyl)-2,4-di(pyridin-2-yl)-3,7-diazabicyclo[3.3.1]nonane-3,7-diyl}bis(-methylene))dipicolinic acid),
H2dedpa (1,2-[{6-(carboxy)-pyridin-2-yl}-methylamino]ethane),
H2macropa (6-(1,4,10,13-tetraoxa-7,16-diazacyclooctadecan-N,N'-methyl)picolinic acid), H5decapa (N,N"-bis(6-carboxy-2-pyridylmethyl)-diethylenetriamine-N,N',N"-triacetic acid),
H6phospa (N,N'-(methylenephosphonate)-N,N'-[6-(methoxycarbonyl)pyridin-2-yl]-methyl-1,2-diaminoethane), HP-D03A (Hydroxypropyltetraazacyclododecanetriacetic acid), and
porphyrin. Among them, the preferred is a compound represented by the following formula (A).

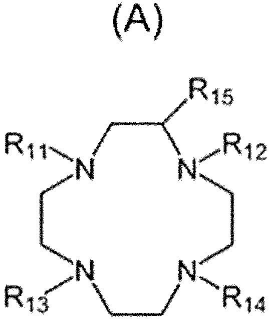

wherein, in formula (A), R₁₁, R₁₂, R₁₃ and R₁₄ are each independently a group consisting of -(CH₂)ₚCOOH, - (CH₂)ₚC₅H₄N, - (CH₂)ₚPO₃H₂, - (CH₂)ₚCONH₂ or -CH (COOH) - (CH₂)ₚCOOH, preferably R₁₁, R₁₃ and R₁₄ are each independently - (CH₂)ₚCOOH, - (CH₂)ₚC₅H₄N, - (CH₂)ₚPO₃H₂ or - (CH₂)ₚCONH₂, R₁₂ is a group consisting of -(CH₂)ₚCOOH, - (CH₂)ₚC₅H₄N, - (CH₂)ₚPO₃H₂, - (CH₂)ₚCONH₂ or -CH(COOH) - (CH₂)ₚCOOH, R₁₅ is a hydrogen atom, and p is an integer of not less than 0 and not more than 3.

The compound represented by formula (A) is preferably a compound containing a structure derived from 1,4,7,10-tetraazacyclododecane-1,4,7,10-tetraacetic acid (DOTA) or a derivative thereof. Specifically, the compound can contain, in its structure, a structure derived from one chelating agent selected from the group consisting of
DOTA (1,4,7,10-Tetraazacyclododecane-1,4,7,10-tetraacetic acid),
DOTMA ((1R,4R,7R,10R)-α,α',α",α‴-tetramethyl-1,4,7,10-tetraazacyclododecane-1,4,7,10-tetraacetic acid),
DOTAM (1,4,7,10-tetrakis(carbamoylmethyl)-1,4,7,10-tetraazacyclododecane),
DOTPA (1,4,7,10-tetraazacyclododecane-1,4,7,10-tetrapropionic acid),
1,4,7,10-tetrakis(pyridin-2-ylmethyl)-1,4,7,10-tetraazacyclododecane (Lpy),
DOTA-GA (α-(2-Carboxyethyl)-1,4,7,10-tetraazacyclododecane-1,4,7,10-tetraacetic acid),
DOTP (((1,4,7,10-Tetraazacyclododecane-1,4,7,10-tetrayl)tetrakis(methylene))tetraphosphonic acid),
DOTMP (1,4,7,10-Tetraazacyclododecane-1,4,7,10-tetrakis(methylenephosphonic acid)),
DOTA-4AMP (1,4,7,10-tetraazacyclododecane-1,4,7,10-tetrakis(acetamidomethylenephosphonic acid)), and D02P (Tetraazacyclododecane dimethanephosphonic acid).

More preferred are compounds represented by the following formulas (A-1) to (A-6). The chelating agent to be used in the radioconjugate in the present invention is further preferably DOTA-GA (compound represented by formula (A-6)).

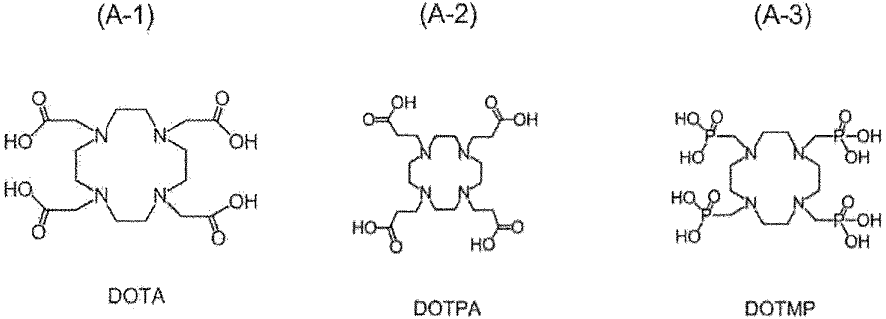

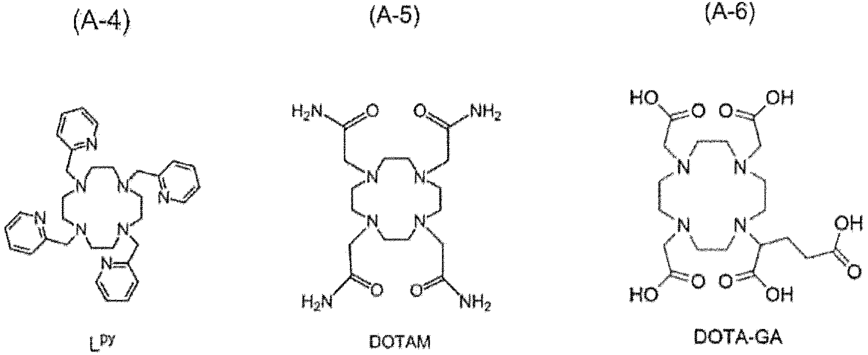

The chelating agent used in the present invention may be directly covalently linked to the polypeptide, or covalently linked to the polypeptide via linker (L). Therefore, in the radioconjugate of the present invention, some groups in the compound of the aforementioned chelating agent may be substituted by a group that can form a covalent bond with the polypeptide (direct bond) or linker (L). For example, when the chelating agent to be used in the present invention is a compound represented by formula (A), R₁₂ or R₁₅ may be substituted by a group that can form a covalent bond with the polypeptide (direct bond) or linker (L). Preferably, when R₁₂ is substituted by a group that can form a covalent bond with the polypeptide (direct bond) or linker (L), then R₁₅ is a hydrogen atom, and when R₁₂ is a group consisting of -(CH₂)ₚCOOH, -(CH₂)ₚC₅H₄N, -(CH₂)ₚPO₃H₂, - (CH₂)ₚCONH₂, or -CH (COOH)-(CH₂)ₚCOOH, then R₁₅ is substituted by a group that can form a covalent bond with the polypeptide (direct bond) or linker (L) (e.g., - (CH₂)ₚC₆H₄-NCS).

Examples of the covalent bond between the chelating agent and the polypeptide (direct bond) or linker (L) include a carbon-carbon bond, an amide bond, an ether bond, an ester bond, a thiourea bond, a thioether bond, a thio ester bond and the like.

The linking between the chelating agent and the polypeptide (direct bond) or linker (L) can be formed, for example, by the reaction of an N-hydroxysuccinimide ester (NHS) group of the following formula (A-7) or (A-8), a 2,6-dioxotetrahydro-2H-pyranyl group of the following formula (A-9), or an isothiocyanate group of DOTA-Bn-SCN (S-2-(4-Isothiocyanatobenzyl)-1,4,7,10-tetraazacyclododecane-1,4,7,10-tetraacetic acid), with an amino group of the polypeptide (direct bond) or linker (L) .

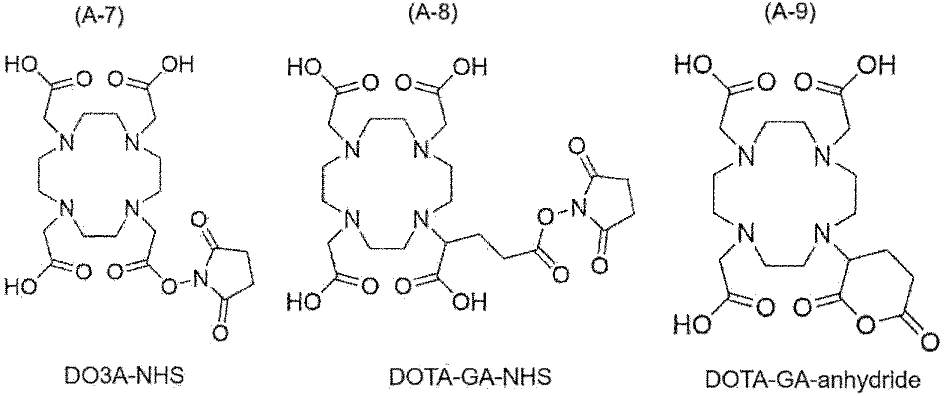

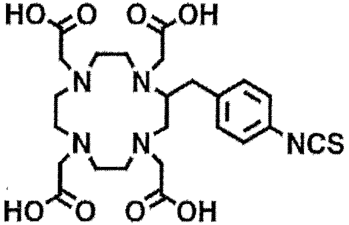

Linker (L) is not particularly limited as long as it can link the chelating agent and the polypeptide in the radioconjugate in the present invention. Linker (L) used in present invention is not particularly limited, and examples thereof include a substituted or unsubstituted alkyl group, a substituted or unsubstituted heteroalkyl group, a polyethylene glycol (PEG) group, a peptide, a sugar chain, a disulfide group, an amide group, combinations thereof and the like.

In the present specification, linker (L) is a general term for linkers used for the connection of a polypeptide and a chelating agent, and includes peptide linker (L₁) and chelate linker (L₂). Peptide linker (L₁) is not particularly limited as long as it can be linked to the amino acid residue of the polypeptide, and chelate linker (L₂) is not particularly limited as long as it can be introduced into the functional group of the chelating agent.

Linker (L) used in the present invention may contain a binding site formed by a click reaction, and preferably, peptide linker (L₁) and chelate linker (L₂) are linked by a click reaction. The click reaction may be a reaction that proceeds between an azide group and an alkyne (for example, Huisgen reaction), or Diels-Alder reaction that proceeds between an alkene and a diene.

Further, the linker may contain a substrate sequence site of a renal brush border membrane enzyme (lysine-glycine-phenylalanine, glycine-lysine, phenylalanine-lysine) or an albumin binding site, as a functional site.

For the substrate sequence site of a renal brush border membrane enzyme, the description in WO 2019/065774 can be referred to as appropriate.

The structure of the albumin binding site includes, for example, structures derived from one or more of γ-glutamic acid, substituted or unsubstituted phenylbutyric acids, lipids, hematin, bilirubin, clofibric acid, clofibrate, carotenoids, compounds with a steroid skeleton, compounds with an ibuprofen skeleton, straight or branched chain and saturated or unsaturated hydrocarbons having 13 to 20 carbon atoms, cyanine dyes, dyes with a sulfonic acid group, diazo dyes, pentamethine cyanine dyes, blue dextran, bromocresol green, and Evans blue and derivatives thereof, or the structures described in WO 2005/117984, WO 2010/127336, or US 2010/0172844. In addition to or instead of these, peptides capable of binding to albumin (for example, the peptides described in WO 2007/106120, etc.) can also be used as an albumin binding site.

In the radioconjugate in the present invention, the polypeptide and the chelating agent are linked directly or via linker (L), preferably, one molecule of the chelating agent is conjugated to the polypeptide.

Examples of the radioconjugate in the present invention include a conjugate in which Physalamine or Somatostatin and a chelate complex are linked directly or via linker (L), or Oxodotreotide or Edotreotide labeled with a radionuclide.

### (1-4) Production method of radioactive peptide (including radioconjugate)

The polypeptide of the radioactive polypeptide in the present invention may be synthesized, for example, by using commercially available Physalamine, or by sequentially linking amino acids protected with an amino-protecting group (for example, Fmoc group etc.) from the C-terminal side on a solid support using an automatic peptide synthesizer.

The amino group can be deprotected by a method known to those skilled in the art (for example, in the case of an Fmoc group, using 20% piperidine/1-methyl-2-pyrrolidinone, etc.) to produce a polypeptide. A chelating agent and linker (L) are introduced into the polypeptide before and after this deprotection.

The radionuclide can be introduced by an isotope exchange reaction, or a substitution reaction of an arbitrary substituent with a radionuclide, or by complexing a radionuclide with a chelating agent. The radionuclide used here is preferably used in an ionizable form, and more preferably in an ionic form, from the aspect of the complex formation efficiency.

While the order of addition of the radionuclide is not limited, the radioconjugate in the present invention can be produced by two steps: a conjugation step of conjugating a chelating agent and a polypeptide, and a complex formation step of forming a complex from a metal radionuclide and a chelating agent. The conjugation step may be performed before the complex formation step or after the complex formation step.

In the complex formation step, for example, the method described in WO 2021/033530 can be used, and a metal radionuclide is chelated with a chelating agent (complex formation). The metal radionuclide used here is preferably used in an ionizable form, and more preferably in an ionic form, from the aspect of the complex formation efficiency. In the complex formation step, the order of addition of the metal radionuclide to the chelating agent is not limited as long as the complex can be formed with the metal radionuclide. For example, a solution in which radioactive metal ions are dissolved in a solvent mainly composed of water can be used as a metal radionuclide.

The complex formation reaction is preferably carried out in a solvent. As the solvent, for example, water or a buffer can be used. The solvent may be contain a water-soluble organic solvent.

As water, for example, distilled water and ion-exchanged water can be used.

As the buffer, acetic acid and a salt thereof, phosphoric acid and a salt thereof, trishydroxymethylaminomethane buffer (Tris buffer), 4-(2-hydroxyethyl)-1-piperazine ethanesulfonate buffer (HEPES buffer), tetramethylammonium acetate buffer and the like can be used.

As the water-soluble organic solvent, for example, polar solvents such as protic solvents (e.g., methanol, ethanol etc.), protic solvents (e.g., acetonitrile, N,N-dimethylformamide, tetrahydrofuran, dimethyl sulfoxide, acetone etc.), and the like are preferably used. Among them, it is more preferable to use at least one selected from acetonitrile, N,N-dimethylformamide, dimethyl sulfoxide and ethanol as the water-soluble organic solvent, from the viewpoint of allowing the complex formation reaction to proceed favorably.

While the amount of the solvent is not particularly limited, from the aspect of practicality in the production step, it is realistically not less than 0.01 mL and not more than 100 mL at the start of the complex formation reaction. The concentration of the chelating agent in the reaction solution is preferably not less than 1 µmοl/L and not more than 1000 µmοl/L at the start of the complex formation reaction, more preferably not less than 10 µmοl/L and not more than 900 µmol/L, still more preferably not less than 30 µmοl/L and not more than 600 µmol/L, even more preferably not less than 50 µmοl/L and not more than 500 µmοl/L, from the aspect of the yield of the desired radioactive metal complex. The concentration of the radioactive metal ion in the reaction solution is preferably not less than 1 nmol/L and not more than 10000 nmol/L at the start of the complex formation reaction, more preferably not less than 10 nmol/L and not more than 5000 nmol/L, still more preferably not less than 50 nmol/L and not more than 3000 nmol/L, even more preferably not less than 100 nmol/L and not more than 2000 nmol/L, from the aspect of the yield of the desired radioactive metal complex.

While the pH of the reaction solution can be changed as appropriate depending on the physical properties of the radioactive metal, chelating agent and buffer used, it is preferably not less than 2.0 and not more than 7.0, more preferably not less than 4.5 and not more than 6.5, still more preferably not less than 5.0 and not more than 6.0.

The temperature of the complex formation reaction may be, for example, room temperature (25°C) or heating conditions. To simultaneously achieve suppression of decomposition of the chelating agent and improvement of complex formation efficiency, it is preferably not less than 30°C and not more than 80°C, more preferably not less than 50°C and not more than 80°C. Provided that the reaction temperature is as described above, the reaction time is preferably not less than 15 minutes and not more than 150 minutes, more preferably not less than 30 minutes and not more than 120 minutes.

The obtained radioconjugate may be used as it is or purified using a filtration filter, a membrane filter, a column filled with various fillers, chromatography or the like.

Even when the conjugation step is performed before the complex formation step, the radioconjugate of the present invention can be produced in the same manner as when the conjugation step is performed after the complex formation step described above.

### (2) Radiopharmaceutical composition

The radiopharmaceutical composition of the present invention refers to a composition that contains the above-mentioned radioactive polypeptide (including radioconjugate) as an active ingredient and is in a form suitable for in vivo administration to a subject (hereinafter, "the radiopharmaceutical composition of the present invention" is also referred to as "the radiopharmaceutical of the present invention"). The radiopharmaceutical may be, for example, the radioactive polypeptide produced by the method shown in the aforementioned (1-4) as it is, or can be produced by purifying same and dissolving same in a solvent containing water, that is, a solvent mainly containing water and approximately isotonic with the living body (e.g., physiological saline). In this case, the radiopharmaceutical is preferably in the form of an aqueous solution.

When the radionuclide is ⁸⁹Zr, the radioactivity concentration of the radioactive polypeptide as an active ingredient is preferably not less than 10 MBq/mL in the radiopharmaceutical of the present invention immediately after production, more preferably not less than 100 MBq/mL, still more preferably not less than 1000 MBq/mL, and it is preferably not more than 3000 MBq/mL, and it is preferably within the range of 10 to 3000 MBq/mL, more preferably within the range of 100 to 3000 MBq/mL, still more preferably within the range of 1000 to 3000 MBq/mL. When the radionuclide is ²²⁵Ac, the radioactivity concentration of the radioactive polypeptide is preferably not less than 0.1 MBq/mL and not more than 300 MBq/mL in the radiopharmaceutical of the present invention, and it is more preferably within the range of 0.1 to 300 MBq/mL.

A solubilizing agent is used in the radiopharmaceutical of the present invention to prevent the radioactive polypeptide as an active ingredient from adsorbing to a container or administration equipment made of glass, plastic or the like.

The solubilizing agent to be used in the present invention is a solubilizing agent having an ethylene glycol group, and examples thereof include polysorbates such as polysorbate 20, polysorbate 80 (hereinafter referred to as PS80) and the like; macrogols such as macrogol 4000 (hereinafter referred to as MG4), macrogol 300 and the like; and polyoxyethylene hydrogenated castor oils. Among them, it is preferably polysorbate (also known as polyoxyethylene sorbitan fatty acid ester) or macrogol (also known as polyethylene glycol), and from the aspect of easy purchase, it is more preferably selected from the group consisting of PS80, polysorbate 20 and MG4.

The solubilizing agent is contained in the radiopharmaceutical of the present invention in an amount of preferably not less than 0.001 w/v%, more preferably not less than 0.01 w/v%, and preferably not more than 10.00 w/v%, more preferably not more than 0.5 w/v%, and preferably within the range of 0.001 to 10.00 w/v%, more preferably within the range of 0.01 to 0.5 w/v%.

The radiopharmaceutical of the present invention contains a stabilizer. The stabilizer suppresses the decrease in the radiochemical purity of the radioactive polypeptide over time due to oxidation reactions. And, unexpectedly, the decrease in radiochemical purity over time is suppressed more than when no solubilizing agent is contained.

The stabilizer to be used in the present invention is selected from the group consisting of sulfur-containing amino acids or salts thereof, sulfur-containing amino acid derivatives or salts thereof, and polysaccharides.

Examples of the sulfur-containing amino acid include cysteine, methionine, homocysteine and the like.

Examples of the sulfur-containing amino acid derivative include peptides containing a sulfur-containing amino acid, such as glutathione and the like, or amino acid derivatives such as cysteine ethyl ester.

Examples of the polysaccharide include disaccharides such as sucrose, lactose, maltose, trehalose and the like; and trisaccharides such as raffinose, maltotriose and the like.

Among them, the stabilizer is preferably selected from the group consisting of methionine, cysteine, glutathione, and sucrose, since they have an excellent suppressive effect on the decrease in the radiochemical purity over time.

The stabilizer is contained in the radiopharmaceutical of the present invention in an amount of preferably not less than 0.01 mg/mL, more preferably not less than 0.1 mg/mL, and preferably not more than 2000 mg/mL, more preferably not more than 200 mg/mL, still more preferably not more than 50 mg/mL, and preferably within the range of 0.01 to 2000 mg/mL, more preferably within the range of 0.01 to 200 mg/mL, still more preferably within the range of 0.1 to 50 mg/mL.

The radiopharmaceutical of the present invention preferably contains ethanol from the aspect of solubilization of the polypeptide. Ethanol is contained in the radiopharmaceutical of the present invention in an amount of preferably not less than 0.01 v/v%, more preferably not less than 0.1 v/v%, and preferably not more than 20 v/v%, more preferably not more than 10 v/v%, and preferably within the range of 0.01 to 20 v/v%, more preferably within the range of 0.1 to 10 v/v%.

As mentioned above, the radiopharmaceutical of the present invention is preferably in the form of an aqueous solution, and from the viewpoint of maintaining radiochemical purity, as described above, it is more preferably in the form of a buffer with a buffering capacity from acidic to near neutral. The pH is preferably 3.0 to 8.0. As the buffer, any buffer commonly used in radiopharmaceuticals can be used, and examples thereof include, but are not limited to, physiological saline; buffers such as acetic acid-sodium acetate buffer (hereinafter referred to as AA buffer), ammonium acetate buffer, phosphate buffer, phosphate-buffered physiological saline, trishydroxymethylaminomethane buffer (Tris buffer), 4-(2-hydroxyethyl)-1-piperazine ethanesulfonate buffer (HEPES buffer), tetramethylammonium acetate buffer and the like. AA buffer is preferably used. AA buffer is composed of acetic acid and sodium acetate.

The radiopharmaceutical of the present invention may further contain other pharmaceutically acceptable components as necessary. An effective amount of the radiopharmaceutical is orally or parenterally, for example, intravenously, subcutaneously, intraperitoneally, intramuscularly, or the like, administered to a living body, and is used for treatment of cancer, diagnosis of cancer, detection of a lesion, or the like.

As used herein, the subject of administration is a human, or an animal such as mouse, rat, monkey, guinea pig, chimpanzee, sheep, goat, dog, cat, swine, bovine, horse or the like, but is not particularly limited. Preferred is a human.

Cancer is a preferred target disease. The type of cancer to be treated, diagnosed or detected in the present invention is not particularly limited, but examples include salivary gland cancer, ovarian cancer, bladder cancer, biliary tract cancer, stomach cancer, liver cancer, and breast cancer. Furthermore, cancer may be at any stage, localized or metastatic, or primary or recurrent.

As used herein, the "effective amount" is an amount that can afford useful diagnostic or therapeutic effects in a subject of administration. The effective amount to be administered to a subject varies depending on the type of subject, body weight of the subject, dosage form (tablet, injection, etc.) and route (oral administration, parenteral administration, etc.) of administration, severity of disease (cancer, etc.), and the like. Physicians and veterinarians can consider these factors and determine the appropriate effective amount.

When the radiopharmaceutical of the present invention thus stabilized is stored at room temperature, the active ingredient (radioactive polypeptide) has a radiochemical purity above a certain level, at a time point after a period of one to five times the half-life of the radionuclide constituting the radiopharmaceutical has passed. Preferably, the difference between the radiochemical purity immediately after production and the radiochemical purity at a time point after a period of one to five times the half-life from the end of production has passed at room temperature is not more than 20%, more preferably not more than 10%.

If the above-mentioned radionuclide is ⁸⁹Zr, the difference between the radiochemical purity immediately after production and the radiochemical purity when stored at room temperature for 7 days after the end of production is preferably not more than 20%, more preferably not more than 10%. If the radionuclide is ²²⁵Ac, the difference between the radiochemical purity immediately after production and the radiochemical purity when stored at room temperature for 14 days after the end of production is preferably not more than 20%, more preferably not more than 10%.

Here, "room temperature" in the present specification preferably refers to "ambient temperature" as defined in the Japanese Pharmacopoeia, and specifically is 15 to 25°C.

The radiochemical purity refers to the percentage of the radioactivity (count) of the peak corresponding to the radioactive polypeptide relative to the total radioactivity (count) detected when a sample containing the radioactive polypeptide is analyzed using a commercial radiation detector. For radiochemical purity analysis, high-performance liquid chromatography or thin layer chromatography can be used, and high-performance liquid chromatography is preferably used.

Examples of the high-performance liquid chromatography method include a method using reverse-phase high-performance liquid chromatography (RP-HPLC).

As stationary phase, for example, a column packed with high-purity spherical silica gel (particle size 3 to 5 µm) to which an alkyl group such as a butyl group and an octadecyl group is bonded is used. As such columns, InertSustainBio C18 (manufactured by GL Science), Protenavi C4 (manufactured by OSAKA SODA), Triart C18, and Bio C4 (manufactured by YMC) are commercially available.

As mobile phase, for example, a mixture of acetonitrile and acetic acid-sodium acetate buffer (pH 6 to 7) can be used, and the column is preferably under conditions where the acetonitrile concentration in the mobile phase is gradually increased relative to the acetic acid-sodium acetate buffer.

The temperature may be at room temperature, or under heating to 30 to 80°C.

This can reduce the half width of the peak of the radioactive peptide, which is the active ingredient, to not more than 0.5. Thus, the radioactive polypeptide and its radioactive impurities (for example, radioactive impurities derived from the oxidized form with the thioether group structure) can be easily separated, and as a result, the radiochemical purity can be evaluated more accurately.

The radiopharmaceutical of the present invention can be used for radionuclide therapy for cancer by selecting a radionuclide that has a therapeutic effect, specifically a radionuclide that emits α rays or a nuclide that emits β rays (preferably ²²⁵Ac, ⁹⁰Y, ¹⁷⁷Lu, more preferably ²²⁵Ac). In this radionuclide therapy, the radiopharmaceutical of the present invention is administered by intravenous injection or orally to cause accumulation of the radioconjugate of the present invention in a lesion site such as a cancer primary lesion or metastatic lesion, and cancer cells at the lesion site are destroyed by the radiation emitted from the radionuclide. The amount to be administered and dose of the radiopharmaceutical of the present invention is appropriately determined by the efficacy of the active ingredient, the mode and route of administration, the stage of disease progression, the body type, body weight and age of the patient, and the type and amount of the therapeutic drug to be used in combination for other diseases.

In addition, by selecting a radionuclide that emits positron or a radionuclide that emits γ rays (preferably ¹¹¹In, ⁶⁷Ga, ⁶⁸Ga, ⁸⁹Zr, more preferably ⁸⁹Zr) as the radionuclide, it can be used for cancer diagnosis or lesion detection. A radiopharmaceutical using a radionuclide that emits positron can be preferably used for PET (Positron Emission Tomography) examination, and a radiopharmaceutical using a radionuclide that emits γ-rays can be preferably used for SPECT (Single Photon Emission Computed Tomography) examination. This may also be used in combination with cancer diagnosis or lesion detection in the aforementioned radionuclide therapy for cancer. When the composition of the present invention is used as a radiopharmaceutical for cancer diagnosis, the composition may be used for diagnosis before performing radionuclide therapy for cancer, or may be used for diagnosis after performing radionuclide therapy for cancer. By using the radiopharmaceutical for diagnosis before conducting radionuclide therapy for cancer, selection of treatment can be performed based on whether or not to perform radionuclide therapy for cancer using the radiopharmaceutical of the present invention. By using the radiopharmaceutical for diagnosis after conducting radionuclide therapy for cancer, moreover, whether or not radionuclide therapy for cancer using the radiopharmaceutical of the present invention is effective can be judged, and a treatment plan including an increase or a decrease of dose and the like can be optimized.

The above embodiments of the present invention encompass the following technical ideas.
[1] A stabilized radiopharmaceutical composition comprising
   a polypeptide labeled with a radionuclide and having a group selected from the group consisting of a thioether group and a thiol group, as an active ingredient,
   a solubilizing agent having an ethylene glycol group,
   a solvent comprising water, and
   a stabilizer selected from the group consisting of a sulfur-containing amino acid or a salt thereof, a sulfur-containing amino acid derivative or a salt thereof, and a polysaccharide.
[2] The stabilized radiopharmaceutical composition of the aforementioned [1], wherein the stabilizer is selected from the group consisting of methionine, cysteine, glutathione and sucrose.
[3] The stabilized radiopharmaceutical composition of the aforementioned [1] or [2], wherein the solubilizing agent is a polysorbate or a macrogol.
[4] The stabilized radiopharmaceutical composition of any one of the aforementioned [1] to [3], further comprising ethanol.
[5] The stabilized radiopharmaceutical composition of any one of the aforementioned [1] to [4], wherein the solvent comprises physiological saline and/or buffer.
[6] The stabilized radiopharmaceutical composition of any one of the aforementioned [1] to [5], wherein the radionuclide is selected from the group consisting of ⁸⁹Zr, ²²⁵Ac, ¹⁷⁷Lu, ¹¹¹In, ⁹⁰Y, ⁶⁷Ga and ⁶⁸Ga.
[7] The stabilized radiopharmaceutical composition of any one of the aforementioned [1] to [6], wherein the polypeptide has a molecular weight of 500 to 10000.
[8] The stabilized radiopharmaceutical composition of any one of the aforementioned [1] to [7], wherein the polypeptide labeled with a radionuclide and having a group selected from the group consisting of a thioether group and a thiol group is a conjugate comprising a complex formed from a chelating agent and a radionuclide, and a polypeptide having a group selected from the group consisting of a thioether group and a thiol group.
[9] The stabilized radiopharmaceutical composition of the aforementioned [8], wherein the chelating agent is a ligand compound represented by the following formula (A):

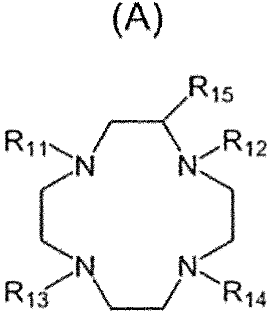

wherein, in formula (A), R₁₁, R₁₂, R₁₃ and R₁₄ are each independently a group consisting of -(CH₂)ₚCOOH, - (CH₂)ₚC₅H₄N, -(CH₂)ₚPO₃H₂, -(CH₂)ₚCONH₂ or -CH (COOH)-(CH₂)ₚCOOH, R₁₅ is a hydrogen atom, and p is an integer of not less than 0 and not more than 3.

### [Example]

The present invention is described in more detail in the following by way of Production Examples and Experimental Examples. However, the present invention is not limited by these examples. The degree of suppression of oxidation reaction can be confirmed by radiochemical purity.

### Production Examples 1 to 3

A radioactive preparation containing a ⁸⁹Zr-labeled product of DOTAGA-Physalaemin (structure shown below; molecular weight 1723.92) (hereinafter to be also referred to as ⁸⁹Zr-labeled product) was prepared by the following procedures (1) to (17).

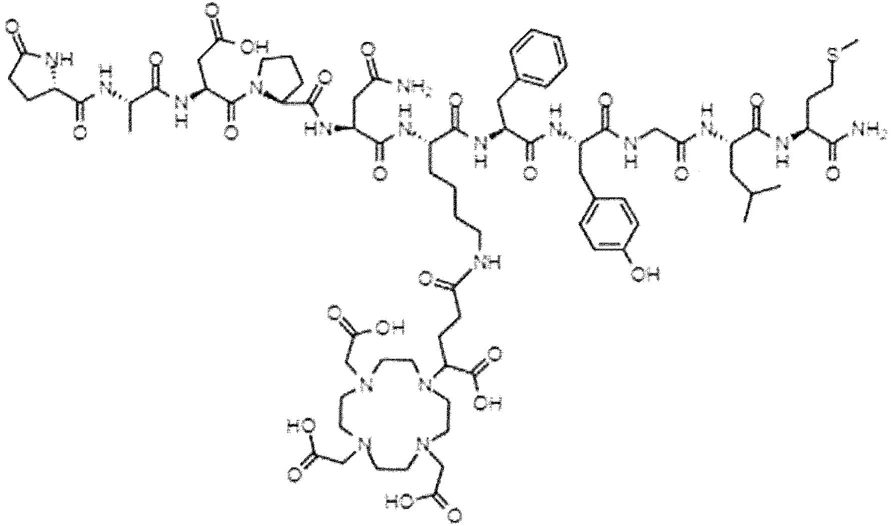

(1) Into Type I Plus vial (2R) (manufactured by SCHOTT), a 1.0 mol/L hydrochloric acid solution containing ⁸⁹Zr ion (hereinafter to be referred to as ⁸⁹Zr solution) was dispensed as a radioactive metal source, and the radioactivity was measured. The amount of the ⁸⁹Zr solution used and the measured radioactivity (charged radioactivity) are shown in Table 3.
(2) The vial of the aforementioned (1) was tightly stoppered, and heated to 110°C, and the solvent was evaporated for about 40 min under an Ar stream.
(3) To the vial of the aforementioned (2), 100 µL of 0.1 mol/L hydrochloric acid was added.
(4) To the vial of the aforementioned (3), 50 µL of 300 mmol/L gentisic acid-0.78 mol/L acetic acid-sodium acetate buffer (pH5.5) was added.
(5) To the vial of the aforementioned (4), 75 µL of 2 mmol/L DOTAGA-Physalaemin-dimethyl sulfoxide solution was added.
(6) To the vial of the aforementioned (5), 75 µL of dimethyl sulfoxide was added.
(7) The vial of the aforementioned (6) was tightly stoppered with a rubber stopper and an aluminum cap, and was stirred using a vortex.
(8) The vial of the aforementioned (7) was warmed to 70°C to start the labeling reaction (60 min).
(9) The solution of the aforementioned (8) was injected into HPLC and purified. The HPLC conditions for purification are as follows.

### HPLC conditions for purification

Detector: ultraviolet absorption photometer (measurement wavelength: 254 nm), radiation detector (Gabi Star, manufactured by Raytest)
Column: InertSustainBioC18 HP 3ur 3.5 um, 4.6 x 100mm, manufactured by GL Sciences
Column temperature: not set
Flow rate: 0.5 mL per minute
Mobile phase A: 5 mmol/L acetic acid-ammonium acetate buffer (pH6.9)
Mobile phase B: acetonitrile
Mobile phase delivery: The concentration gradient was controlled by changing the mixing ratio of mobile phase A and mobile phase B as shown in Table 1.

**[Table 1]**

| Time after injection (min) | Mobile phase A (vol%) | Mobile phase B (vol%) |
|---|---|---|
| 0.0-5.0 | 90 | 10 |
| 5.0-8.0 | 90→70 | 10→30 |
| 8.0-25.0 | 70 | 30 |
| 25.0-25.1 | 70→60 | 30→40 |
| 25.1-30.0 | 60→50 | 40→50 |
| 30.0-30.1 | 50→90 | 50→10 |
| 30.1-35.0 | 90 | 10 |

(10) The HPLC-purified fractions were diluted by adding 10 mL of water for injection, and passed through Sep pak tC18 Light (manufactured by Waters). The Sep pak tC18 Light was conditioned in advance by passing anhydrous ethanol (5 mL) and then water for injection (10 mL).
(11) Water for injection (10 mL) was passed through the column of the aforementioned (10).
(12) 70% EtOH (0.5 mL) was passed through the column of the aforementioned (11). The eluate was collected in a colorless glass vial with a rubber stopper (capacity 6 mL), and the radioactivity was measured. The measurement results of the radioactivity recovered from the simple solid phase extraction column are shown in Table 3.
(13) The vial of the aforementioned (12) was tightly stoppered with an aluminum cap, warmed to 70°C, and dried under an Ar stream.
(14) A diluting solvent was added to the vial of the aforementioned (13) so that the solution had the desired radioactivity concentration to produce the desired preparations (entries 1 to 15). The type of the diluting solvent and the radioactivity concentration are shown in Table 4.
(15) The preparations of the aforementioned (14) were tested in the following Experimental Examples 1 to 4, and subjected to HPLC analysis. The HPLC conditions for analysis are as follows.

### HPLC conditions for analysis

Detector: ultraviolet absorptiometer (measurement wavelength: 254 nm), radiation detector (Gabi Star, manufactured by Raytest)
Column: InertSustainBioC18 HP 3ur 3.5 µm, 4.6 × 100 mm, manufactured by GL Sciences
Column temperature: 60°C
Mobile phase A: 5 mmol/L acetic acid-ammonium acetate buffer (pH6.9)
Mobile phase B: acetonitrile
Mobile phase delivery: The concentration gradient was controlled by changing the mixing ratio of mobile phase A and mobile phase B as shown in Table 2. Flow rate: 1.0 mL per minute

**[Table 2]**

| Time after injection (min) | Mobile phase A (vol%) | Mobile phase B (vol%) |
|---|---|---|
| 0.0-5.0 | 90 | 10 |
| 5.0-8.0 | 90→70 | 10→30 |
| 8.0-15.0 | 70 | 30 |
| 15.0-15.1 | 70→90 | 30→10 |
| 15.1-25.0 | 90 | 10 |

**[Table 3]**

| Production Example | Use | Amount of ⁸⁹Zr solution used (µL) | Charged Radioactivity (MBq) | Radioactivity recovered from simple solid phase extraction column (MBq) | Radiochemical yield (%) |
|---|---|---|---|---|---|
| 1 | Used in Experimental Examples 1 to 4 | 650 | 410 | 293 | 71.5 |
| 2 | Used in Experimental Examples 1 and 4 | 182 | 159 | 77.0 | 48.4 |
| 3 | Used in Experimental Example 3 | 479 | 517 | 320 | 61.9 |

**[Table 4]**

| Production Example | Lot | Formulation composition | Radioactivity concentration (MBq/mL) |
|---|---|---|---|
| 1 | entry 1 | 0.5w/v% PS80-10% ethanol-158mmol/L AA buffer (pH6.5) | 35.52 |
| | entry 2 | 0.5w/v% PS80-10% ethanol-0.5mg/mL gentisic acid-158mmol/L AA buffer (pH6.5) | 35.89 |
| | entry 3 | 0.5w/v% PS80-10% ethanol-50mg/mL ascorbic acid-158mmol/L AA buffer (pH6.5) | 32.56 |
| | entry 4 | 0.5w/v% PS80-10% ethanol-25mg/mL cysteine hydrochloride monohydrate-158mmol/L AA buffer (pH6.5) | 31.45 |
| | entry 5 | 0.5w/v% PS80-10% ethanol-0.65mg/mL methionine-158mmol/L AA buffer (pH6.5) | 28.49 |
| | entry 6 | 0.5w/v% PS80-10% ethanol-50mg/mL fructose-158mmol/L AA buffer (pH6.5) | 28.86 |
| | entry 7 | 0.5w/v% PS80-10% ethanol-50mg/mL sucrose-158mmol/L AA buffer (pH6. 5) | 29.97 |
| | entry 8 | 0.5w/v% MG4-316mmol/L AA buffer (pH6.5) | 34.78 |
| | entry 9 | 0.5w/v% MG4-0.65mg/mL methionine-316mmol/L AA buffer (pH6.5) | 35.89 |
| | entry 10 | 0.5w/v% PS80-10% ethanol-0.5w/v% glutathione-158mmol/L AA buffer (pH6.5) | 28.12 |
| 2 | entry 11 | 10% ethanol-158mmol/L AA buffer (pH6.5) | 37.00 |
| | entry 12 | 158 mmol/L AA buffer (pH6.5) | 38.11 |
| | entry 13 | 0.5w/v% PS80-158mmol/L AA buffer (pH6.5) | 34.04 |
| 3 | entry 14 | 0.5w/v% PS80-10% ethanol-158mmol/L AA buffer (pH6.5) | 1000 |
| | entry 15 | 0.5w/v% PS80-10% ethanol-0.65mg/mL methionine-158mmol/L AA buffer (pH6.5) | 1000 |

### Experimental Example 1 Evaluation of actions of PS80 and ethanol

The radioactive preparations of entries 1 and 11 to 13 prepared in Production Examples 1 and 2 were subjected to HPLC analysis immediately after production and 1, 2, 5, 7 and 9 days after production, and the effects of PS80 and ethanol on the decrease in the radiochemical purity of the ⁸⁹Zr-labeled product over time were examined. The results are shown in Table 5 and Fig.1. In Table 5, "decrease in radiochemical purity at a time point from immediately after production" means the difference between radiochemical purity immediately after production and radiochemical purity at each time point.

**[Table 5]**

| Lot | Solubilizing agent | Ethanol (%) | Stabilizer | Radiochemical purity (%) immediately after production | Decrease (%) in radiochemical purity at a time point from immediately after production | | | | |
|---|---|---|---|---|---|---|---|---|---|
| | | | | | 1 days | 2 days | 5 days | 7 days | 9 days |
| entry 1 | PS80 | 10 | none | 86.13 | - | 10.00 | 28.97 | 40.58 | 47.54 |
| entry 13 | PS80 | 0 | none | 93.98 | 7.98 | - | 55.60 | 68.63 | - |
| entry 11 | none | 10 | none | 94.43 | 0.38 | - | 11.75 | 17.31 | - |
| entry 12 | none | 0 | none | 93.74 | 0.76 | - | 19.17 | 27.19 | - |

The results in Table 5 and Fig.1 show that PS80 exacerbates the decrease in the radiochemical purity of the ⁸⁹Zr-labeled product over time (comparison of entry 12 and entry 13) and that the trend is confirmed even in the presence of ethanol (comparison of entry 1 and entry 11).

The results also show that although ethanol has a certain effect on suppressing the decrease in the radiochemical purity of the ⁸⁹Zr-labeled product over time (comparison of entry 11 and entry 12), its suppressive effect is not sufficient in the presence of PS80 (comparison of entry 1 and entry 13).

### Experimental Example 2 Evaluation of suppressive effects of various reagents (about 37 MBq/mL)

The radioactive preparations of entries 1 to 7 and 10 prepared in Production Example 1 were subjected to HPLC analysis immediately after production and 2, 5, 7 and 9 days after production, and the effects of various reagents on suppressing the decrease in the radiochemical purity of the ⁸⁹Zr-labeled product over time were examined. The results are shown in Table 6 and Fig.2. In Table 6, "decrease in radiochemical purity at a time point from immediately after production" means the difference between radiochemical purity immediately after production and radiochemical purity at each time point.

**[Table 6]**

| Lot | Solubilizing agent | Ethanol (%) | Stabilizer | Radiochemical purity (%) immediately after production | Decrease (%) in radiochemical purity at a time point from immediately after production | | | |
|---|---|---|---|---|---|---|---|---|
| | | | | | 2 days | 5 days | 7 days | 9 days |
| entry 1 | PS80 | 10 | none | 86.13 | 10.00 | 28.97 | 40.58 | 47.54 |
| entry 2 | PS80 | 10 | gentisic acid | 85.14 | 10.33 | 25.92 | 34.97 | 43.92 |
| entry 3 | PS80 | 10 | ascorbic acid | 86.43 | 21.26 | 47.41 | 58.87 | 69.39 |
| entry 4 | PS80 | 10 | cysteine | 87.31 | <0.01 | <0.01 | <0.01 | <0.01 |
| entry 5 | PS80 | 10 | methionine | 86.25 | <0.01 | 0.49 | 0.90 | 1.23 |
| entry 6 | PS80 | 10 | fructose | 86.10 | 11.39 | 33.41 | 42.72 | 52.89 |
| entry 7 | PS80 | 10 | sucrose | 85.62 | 5.14 | 15.99 | 19.76 | 23.40 |
| entry 10 | PS80 | 10 | glutathione | 86.11 | <0.01 | 3.81 | 1.35 | 3.15 |

The results in Table 6 and Fig.2 show that cysteine (entry 4), methionine (entry 5), sucrose (entry 7) and glutathione (entry 10) have good effects on suppressing the decrease in the radiochemical purity of the ⁸⁹Zr-labeled product over time, whereas gentisic acid (entry 2) has little effect on suppressing the decrease in purity, and ascorbic acid (entry 3) and fructose (entry 6) exacerbate the decrease in purity.

### Experimental Example 3 Evaluation of methionine

The radioactive preparations of entries 1, 5, 14 and 15 prepared in Production Examples 1 and 3 were subjected to HPLC analysis immediately after production and 2 days after production, and the effects of methionine on suppressing the decrease in the radiochemical purity of the ⁸⁹Zr-labeled product over time were examined. The results are shown in Table 7. In Table 7, "decrease in radiochemical purity at a time point from immediately after production" means the difference between radiochemical purity immediately after production and radiochemical purity 2 days after production.

**[Table 7]**

| Lot | Solubilizing agent | Ethanol (%) | Stabilizer | Radioactivity concentration (MBq/mL) | Radiochemical purity (%) immediately after production | Decrease (%) in radiochemical purity at a time point from immediately after production |
|---|---|---|---|---|---|---|
| entry 1 | PS80 | 10 | none | 35.52 | 86.1 | 10.0 |
| entry 14 | PS80 | 10 | none | 1000 | 95.7 | 30.3 |
| entry 5 | PS80 | 10 | methionine | 28.49 | 86.3 | <0.01 |
| entry 15 | PS80 | 10 | methionine | 1000 | 96.2 | 6.5 |

The results in Table 7 show that the decrease in the radiochemical purity at a time point from immediately after production was 30.3% under the condition with a radioactivity concentration of 1000 MBq/mL and no stabilizer (entry 14), whereas the decrease in the radiochemical purity at a time point from immediately after production was 6.5% under the condition with methionine added as a stabilizer (entry 15), and that methionine has a good effect on suppressing the decrease in purity.

### Experimental Example 4 Evaluation of other solubilizing agents

The radioactive preparations of entries 13, 8 and 9 prepared in Production Examples 1 and 2 were subjected to HPLC analysis immediately after production and 1, 2, 5, 7 and 9 days after production, and the effects of various stabilizers on suppressing the decrease in the radiochemical purity of the ⁸⁹Zr-labeled product over time were examined. The results are shown in Table 8 and Fig.3. In Table 8, "decrease in radiochemical purity at a time point from immediately after production" means the difference between radiochemical purity immediately after production and radiochemical purity at each time point.

**[Table 8]**

| Lot | Solubilizing agent | Ethanol (%) | Stabilizer | Radiochemical purity (%) immediately after production | Decrease (%) in radiochemical purity at a time point from immediately after production | | | | |
|---|---|---|---|---|---|---|---|---|---|
| | | | | | 1 days | 2 days | 5 days | 7 days | 9 days |
| entry 13 | PS80 | none | none | 93.98 | 7.98 | - | 55.60 | 68.63 | - |
| entry 8 | MG4 (PEG) | none | none | 86.91 | - | 86.91 | 86.91 | 86.91 | 86.91 |
| entry 9 | MG4 (PEG) | none | methionine | 94.13 | - | 3.09 | 5.56 | 5.70 | 6.11 |

The results in Table 8 show that MG4 exacerbates the decrease in the radiochemical purity over time compared to PS80 (comparison of entry 13 and entry 8), but the addition of methionine has a good effect on suppressing the decrease in the radiochemical purity of the ⁸⁹Zr-labeled product over time.

### Production Example 4

A radioactive preparation containing an ²²⁵Ac-labeled product of DOTAGA-Physalaemin (hereinafter to be also referred to as ²²⁵Ac-labeled product) was prepared by the following procedures (1) to (12).
(1) Into a 1.5 mL Eppendorf tube (Protein LoBind Tube, manufactured by Eppendorf), 9.9MBq of a solution containing ²²⁵Ac ion (0.1 mol/L hydrochloric acid aqueous solution, radioactivity concentration 248 MBq/mL, prepared from Rosatom, volume 0.04 mL) (hereinafter to be referred to as ²²⁵Ac solution) was dispensed as a radioactive metal source.
(2) to the Eppendorf tube of the aforementioned (1), 20.6 µL of 0.78 mol/L acetic acid-sodium acetate buffer (pH5.5), 51.4 µL of water for injection, and 48 µL of 2 mmol/L DOTAGA-Physalaemin-dimethyl sulfoxide solution were added, and the mixture was stirred.
(3) The Eppendorf tube of the aforementioned (2) was warmed to 70°C to start the labeling reaction (60 min).
(4) The solution of the aforementioned (3) was diluted by adding 4524 µL of water for injection, and passed through Oasis HLB Plus Light (manufactured by Waters). The Oasis HLB Plus Light was conditioned in advance by passing anhydrous ethanol (5 mL) and then water for injection (10 mL).
(5) The Eppendorf tube used in the aforementioned (3) was washed with water for injection (total 4 mL), and the recovered washing was passed through the column of the aforementioned (4). The procedure of washing the Eppendorf tube and passing the washing through the column was performed twice in total.
(6) 70 v/v% ethanol aqueous solution (0.5 mL) was passed through the column of the aforementioned (5). The eluate (2.9 MBq) was collected in a colorless glass vial with a rubber stopper (capacity 6 ML).
(7) The vial of the aforementioned (6) was tightly stoppered with an aluminum cap, heated to 70°C, and dried under an Ar stream.
(8) To the vial of the aforementioned (7), 368 µL of an aqueous solution containing 1.0 w/v% polysorbate 80 and 20 v/v% ethanol was added.
(9) 100 µL of the solution of the aforementioned (8) was dispensed into each of two colorless glass vials with rubber stoppers (capacity 6 mL) to prepare entry 16 and entry 17.
(10) The radioactivity of the vials of the aforementioned (9) was measured, and each radioactivity concentration was determined (entry 16: 8.1 MBq/mL, entry 17: 9.8 MBq/mL). The radioactivity concentration was calculated by dividing the radioactivity of the vial by 100 µL.
(11) An aqueous solution containing 1.0 w/v% polysorbate 80 and 20v/v% ethanol was added to each vial of the aforementioned (10) so that the solutions had a radioactivity concentration of 4 MBq/mL (entry 16: 102 µL added, entry 17: 145 µL added).
(12) 202 µL of 0.316 mol/L acetic acid-sodium acetate buffer (pH6.5) was added to entry 16 of the aforementioned (11), and 245 µL of 0.316 mol/L acetic acid-sodium acetate buffer (pH6.5) containing 1.3 mg/mL methionine was added to entry 17 of the aforementioned (11), so that the solution had a radioactivity concentration of 2 MBq/mL.

### Experimental Example 5 Evaluation of stabilizing effect of ²²⁵Ac-labeled product

The radioactive preparations of entries 16 and 17 prepared in Production Example 4 were subjected to TLC analysis immediately after production and 7 days and 17 days after production, and the effects of methionine addition on suppressing the decrease in the radiochemical purity of the ²²⁵Ac-labeled product over time were examined. TLC conditions are as follows. The results are shown in Table 9. In Table 9, "decrease in radiochemical purity at a time point from immediately after production" means the difference between radiochemical purity immediately after production and radiochemical purity at each time point.

### TLC conditions

detector: TLC scanner (GITA Star, manufactured by Raytest)
TLC plate: iTLC-SG, developing solvent: acetonitrile/water=1:1

**[Table 9]**

| Lot | Stabilizer | Radiochemical purity (%) immediately after production | Decrease (%) in radiochemical purity at a time point from immediately after production | |
|---|---|---|---|---|
| | | | 7 days | 17 days |
| entry 16 | none | 96.0 | 0.5 | 38.9 |
| entry 17 | methionine | 95.8 | 4.6 | 15.9 |

The results in Table 9 show that methionine suppresses the decrease in the radiochemical purity of the ²²⁵Ac-labeled product over time.

## Claims

1. A stabilized radiopharmaceutical composition comprising
a polypeptide labeled with a radionuclide and having a group selected from the group consisting of a thioether group and a thiol group, as an active ingredient,
a solubilizing agent having an ethylene glycol group,
a solvent comprising water, and
a stabilizer selected from the group consisting of a sulfur-containing amino acid or a salt thereof, a sulfur-containing amino acid derivative or a salt thereof, and a polysaccharide.

2. The stabilized radiopharmaceutical composition according to claim 1, wherein the stabilizer is selected from the group consisting of methionine, cysteine, glutathione and sucrose.

3. The stabilized radiopharmaceutical composition according to claim 1 or 2, wherein the solubilizing agent is a polysorbate or a macrogol.

4. The stabilized radiopharmaceutical composition according to claim 1 or 2, further comprising ethanol.

5. The stabilized radiopharmaceutical composition according to claim 1 or 2, wherein the solvent comprises physiological saline and/or buffer.

6. The stabilized radiopharmaceutical composition according to claim 1 or 2, wherein the radionuclide is selected from the group consisting of ⁸⁹Zr, ²²⁵Ac, ¹⁷⁷Lu, ¹¹¹In, ⁹⁰Y, ⁶⁷Ga and ⁶⁸Ga.

7. The stabilized radiopharmaceutical composition according to claim 1 or 2, wherein the polypeptide has a molecular weight of 500 to 10000.

8. The stabilized radiopharmaceutical composition according to claim 1 or 2, wherein the polypeptide labeled with a radionuclide and having a group selected from the group consisting of a thioether group and a thiol group is a conjugate comprising a complex formed from a chelating agent and a radionuclide, and a polypeptide having a group selected from the group consisting of a thioether group and a thiol group.

9. The stabilized radiopharmaceutical composition according to claim 8, wherein the chelating agent is a ligand compound represented by the following formula (A): 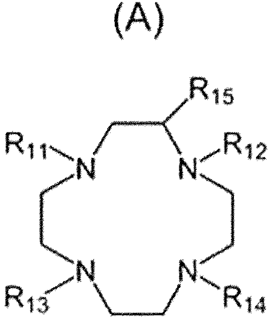 wherein, in formula (A), R₁₁, R₁₂, R₁₃ and R₁₄ are each independently a group consisting of -(CH₂)ₚCOOH, - (CH₂)ₚC₅H₄N, - (CH₂)ₚPO₃H₂, - (CH₂)ₚCONH₂ or -CH (COOH) - (CH₂)ₚCOOH, R₁₅ is a hydrogen atom, and p is an integer of not less than 0 and not more than 3.
